(19) Europäisches Patentamt
European Patent Office
Office européen des brevets

(11) Publication number: **0 450 765 B1**

# (12) EUROPEAN PATENT SPECIFICATION

(45) Date of publication of patent specification: **23.08.95**

(51) Int. Cl.[6]: **C09B 57/00**, C07D 493/04

(21) Application number: **91301785.1**

(22) Date of filing: **04.03.91**

The file contains technical information submitted after the application was filed and not included in this specification

## (54) Polycyclic dyes.

(30) Priority: **06.04.90 GB 9007838**

(43) Date of publication of application: **09.10.91 Bulletin 91/41**

(45) Publication of the grant of the patent: **23.08.95 Bulletin 95/34**

(84) Designated Contracting States: **AT BE CH DE DK ES FR GB GR IT LI NL SE**

(56) References cited:
**EP-A- 133 583**
**EP-A- 2 363 034**

(73) Proprietor: **ZENECA LIMITED**
**15 Stanhope Gate**
**London W1Y 6LN (GB)**

(72) Inventor: **Hall, Nigel**
**35 Newton Drive,**
**Greenmount**
**Bury,**
**Lancashire, BL8 4DH (GB)**
Inventor: **Robinson, Frank**
**32 Springside View,**
**Brandlesholme**
**Bury,**
**Lancashire, BL8 4Lu (GB)**

(74) Representative: **Giles, David Eric et al**
**Intellectual Property Group**
**Zeneca Specialties**
**P.O. Box 42**
**Hexagon House**
**Blackley**
**Manchester M9 8ZS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This specification describes an invention which relates to certain novel polycyclic dyes which have improved dyeing properties.

Example 27 of EP-A-33583 has the structure:

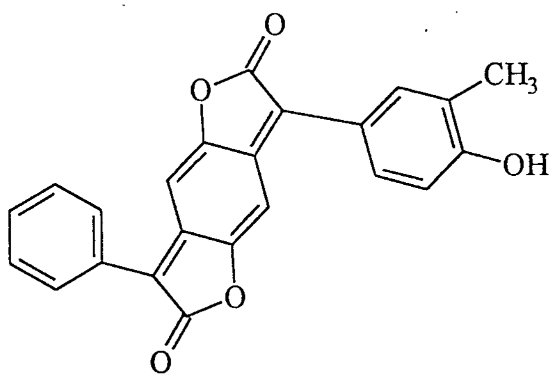

According to the present invention there are provided polycyclic dyes of Formula A:

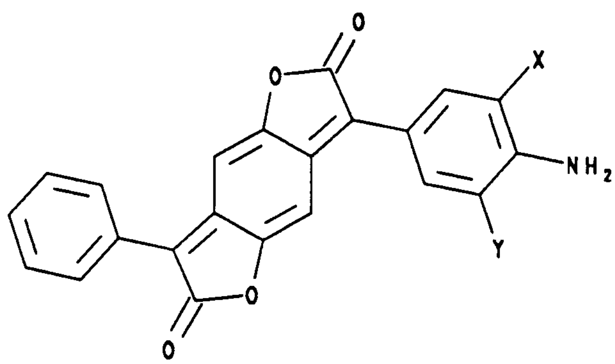

Formula A

wherein:

X is $-C_{2-4}$-alkyl; and Y is $C_{1-4}$-alkyl.

X is preferably ethyl and Y is preferably $C_{1-3}$-alkyl, more preferably $C_{1-2}$-alkyl. It is further preferred that when X is ethyl, Y is ethyl or methyl and especially the latter.

The dyes of the present invention are conveniently prepared by reacting a phenyltartronic acid of Formula B:

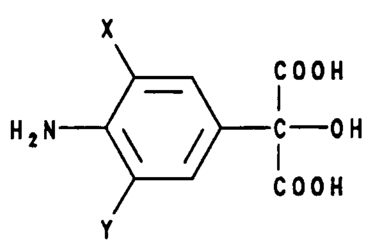

Formula B

wherein X and Y are as defined above with a compound of Formula C:

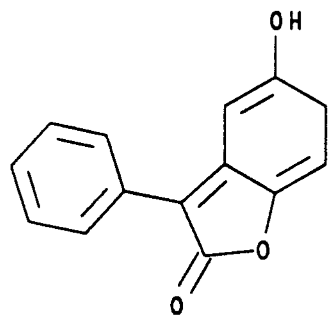

Formula C

and oxidation of the intermediate compound to dehydrogenate the peripheral heterocyclic rings.

The present process may be performed by heating the reactants in a melt but preferably in an acidic medium, preferably an organic acid, and especially an alkanecarboxylic acid such as acetic acid, propionic acid or butyric acid.

The process is preferably performed at a temperature from 50 ° C to 100 ° C, especially 70 ° C to 100 ° C, where a solvent is present, conveniently under reflux. Reaction is preferably continued until all the starting materials are consumed which can take up to 25 hours.

The final oxidation step may be effected by any convenient oxidising agent for dehydrogenating a carbon-carbon single bond, such as a persulphate or hydrogen peroxide.

A compound of Formula C may prepared by reacting mandelic acid with 1,4-dihydroxybenzene.

This reaction is conveniently performed in an acidic medium as described above for the preparation of the compound of Formula A but at a lower temperature in the range 40 ° C to 80 ° C especially from 49 ° C to 75 ° C. It is preferred to perform the process in the presence of a strong acid catalyst. It is especially preferred that the catalyst is sulphuric acid.

The compounds of Formula A described above give blue shades when applied to synthetic fibres, especially polyesters, by disperse dyeing processes, and build up exceptionally well to give strong navy shades for example the dye of Formula A where X is ethyl and Y is methyl has superior build up on polyester to the closest dye disclosed previously in European Patent Application No. 0363034.

Specific examples of preferred dyes of Formula A are shown in Table 1:

Table 1

| X | Y |
|---|---|
| $-C_2H_5$ | $-CH_3$ |
| " | $-C_2H_5$ |
| " | $-(CH_2)_2CH_3$ |
| " | $-CH(CH_3)_2$ |
| " | $-(CH_2)_3CH_3$ |
| " | $-CH_2CH(CH_3)_2$ |
| " | $-CH(CH_3)CH_2CH_3$ |
| " | $-C(CH_3)_3$ |

The invention is further illustrated by the following example in which all parts and percentages are by weight unless otherwise indicated.

Example 1

A mixture of 2.26 parts of 5-hydroxy-2-oxo-3-phenyl-2,3-dihydrobenzofuran, 4.9 parts of 4-amino-3-ethyl-5-methylphenyl tartronic acid (dipotassium salt) and 50 parts glacial acetic acid was stirred under reflux for 12 hours before adding 2.5 parts of ammonium persulphate and continuing heating for a further 1 hour. After cooling to ambient temperature the precipitated solid was filtered, washed acid-free with water, washed with methanol and further purified by column chromatography to give 3-phenyl-7-(4-amino-3-ethyl-5-methylphenyl)-2,6-dioxo-2,6-dihydrobenzo[1:2-b, 4'5-b']difuran. The product dissolved in dichloromethane to give a blue solution with an absorption maximum at 580nm.

When applied to polyester material from an aqueous dispersion it built up well to give navy blue shades with excellent fastness to heat and wash treatments.

Example 2

The procedure of Example 1 was repeated except that the 4.9 parts of 4-amino-3-ethyl-5-methylphenyl tartronic acid (dipotassium salt) was replaced by 4.9 parts of 4-amino-3,5-diethylphenyltartronic acid (dipotassium salt), to give 3-phenyl-7-(4-amino-3,5-diethylphenyl)-2,6-dioxo-2,6-dihydrobenzo[1:2-b, 4:5-b']-difuran. This dissolved in dichloromethane to give a blue solution with an absorption maximum at 584nm.

When applied to polyester material from an aqueous dispersion it built up well to give navy blue shades with excellent fastness to heat and wash treatments.

**Claims**

1. A polycyclic dye of the formula:

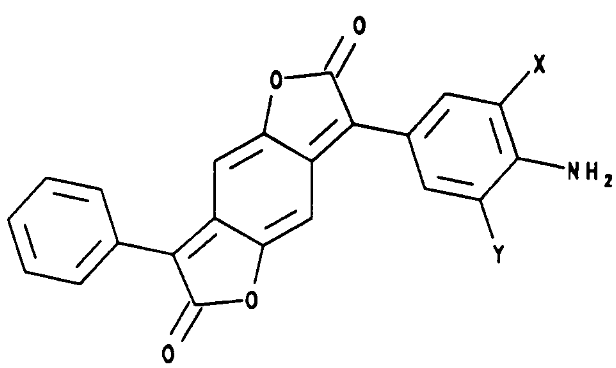

Formula A

wherein:
X is -$C_{2-4}$-alkyl and Y is $C_{1-4}$-alkyl.

2. The polycyclic dye according to claim 1 wherein X is ethyl and Y is methyl.

3. The polycyclic dye according to claim 1 wherein X is ethyl and Y is ethyl.

**Patentansprüche**

1. Polycyclischer Farbstoff mit der Formel:

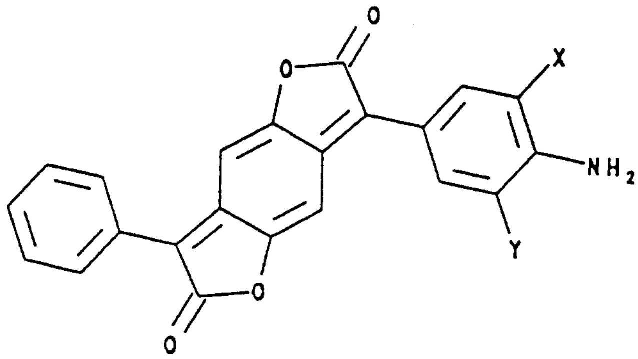

Formel A

in der:
X für $C_{2-4}$-Alkyl und Y für $C_{1-4}$-Alkyl steht.

2. Polycyclischer Farbstoff nach Anspruch 1 wobei X für Ethyl und Y für Methyl steht.

3. Polycyclischer Farbstoff nach Anspruch 1, wobei X für Ethyl und Y für Ethyl steht.

**Revendications**

1. Colorant polycyclique de formule :

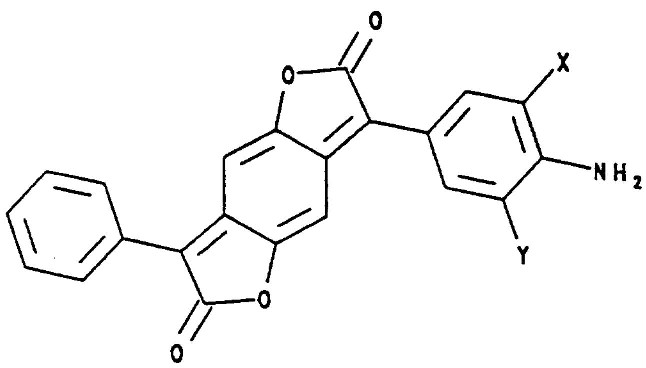

Formule e

dans laquelle :
X est un groupe alkyle en $C_2$ à $C_4$ et Y est un groupe alkyle en $C_1$ à $C_4$.

2. Colorant polycyclique suivant la revendication 1, dans lequel X est un groupe éthyle et Y est un groupe méthyle.

3. Colorant polycyclique suivant la revendication 1, dans lequel X est un groupe éthyle et Y est un groupe éthyle.